# EUROPÄISCHE PATENTSCHRIFT

(11) **EP 0 598 738 B1**
(45) Veröffentlichungstag und Bekanntmachung des Hinweises auf die Patenterteilung: **30.08.1995**
(21) Anmeldenummer: 92912831.2
(22) Anmeldetag: 27.06.1992
(51) Int. Cl.: A61B 3/028

(54) **AUTOMATISCHE, SUBJEKTIV GESTEUERTE REFRAKTIONSEINRICHTUNG**
AUTOMATIC, SUBJECTIVELY CONTROLLED REFRACTION DEVICE
INSTALLATION DE REFRACTION AUTOMATIQUE COMMANDEE SUR LA BASE DE LA METHODE SUBJECTIVE

(30) Priorität: 19.07.1991 DE 4124056
(43) Veröffentlichungstag der Anmeldung: 01.06.1994
(73) Patentinhaber: PREUSSNER, Paul Rolf, Dr., 55131 Mainz (DE)
(72) Erfinder: PREUSSNER, Paul Rolf, Dr., 55131 Mainz (DE)
(86) Internationale Anmeldenummer: DE9200530
(87) Internationale Veröffentlichungsnummer: WO9301744

(56) Entgegenhaltungen:
- EP-A- 0 319 446
- DE-A- 3 314 176
- US-A- 3 861 790

## Beschreibung

### Technisches Gebiet

Bei der Erfindung handelt es sich um ein Gerät, mit dessen Hilfe der Augenarzt oder Optiker die für den jeweiligen Patienten am besten geeigneten Augengläser ermitteln kann.

### Stand der Technik

Die Anpassung von Augengläsern (Brille oder Kontaktlinsen) verläuft nach dem heutigen Stand der Technik im allgemeinen in folgenden Schritten:
Zunächst wird die sogenannte "objektive Refraktion" jedes einzelnen Auges bestimmt. Hierzu gibt es Geräte, die durch die optisch brechenden Medien des Auges ein Bild auf die Netzhaut projizieren und wieder zurück abbilden, so daß der untersuchende Augenarzt an je nach Bauart verschiedenen Parametern ablesen kann, mit welchen Vorsatzlinsen das betreffende Auge korrigiert werden müßte. Neben diesen seit mehreren Jahrzehnten bekannten Geräten sind seit einigen Jahren auch automatische Refraktionsgeräte kommerziell verfügbar, die ebenfalls ein Bild auf die Netzhaut projizieren und die Korrekturgläser des betreffenden Patienten berechnen.

Die Ergebnisse der objektiven Refraktion sind im allgemeinen für die optimale Anpassung einer Brille zu ungenau. Dies hat mehrere Gründe: die optische Achse bei der objektiven Refraktion stimmt meist nicht genau genug mit der subjektiv optimalen Sehachse überein; die Pupille ist bei der objektiven Refraktion meist weiter als physiologischerweise unter Tageslichtbedingungen; der Patient kann durch Akkommodation die Brechkraft seiner Augenlinse unbemerkt verändern; Augengläser, die jedes einzelne Auge für sich optimal korrigieren, sind für den beidäugigen Seheindruck oft nicht optimal.

Aus den genannten Gründen ist ein subjektiver Gläserabgleich zunächst für jedes einzelne Auge, vor allem aber für das Zusammenspiel beider Augen erforderlich. Hierbei werden dem Patienten verschiedene Gläser vorgesetzt, geeignete Sehzeichen projiziert (z.B. 8 4 6 1 7), bestimmte Anweisungen gegeben, etwa: "schauen Sie auf die 6 oder die 8", und Fragen gestellt wie z.B.: "sagen Sie, ob diese Zahlen jetzt unschärfer werden". Für den beidäugigen Abgleich wird oft polarisiertes Licht verwendet, wobei die Polarisationsebenen des rechten und des linken Auges senkrecht aufeinander stehen, so daß der Seheindruck des rechten und des linken Auges getrennt werden können. Auch besteht die Möglichkeit, den Seheindruck beider Augen mit roten und grünen Filtern zu trennen. Die vorgesetzten Gläser sind in einem sogenannten "Phoropter" angeordnet, wodurch ein einfaches und vor allem schnelles Wechseln entweder direkt manuell oder ferngesteuert durch Bedienung einer Tastatur möglich ist.

Der skizzierte Vorgang der "subjektiven Refraktionsbestimmung" ist in der einschlägigen Fachliteratur ausführlich beschrieben und gehört zur Ausbildung des Augenarztes, zum Teil auch des Augenoptikers.

In der Praxis nimmt die Anpassung von Augengläsern einen großen Teil der Arbeitszeit des Arztes in Anspruch. Der Vorgang ist einerseits zu kompliziert, als daß er ohne weiteres durch Hilfspersonal ausgeführt werden könnte, andererseits jedoch logisch klar determiniert, denn es handelt sich im Prinzip mathematisch um ein Approximationsproblem, bei dem diejenigen Augengläser zu finden sind, mit denen der Patient beidäugig subjektiv "am besten sieht".

Die Gläser sind dabei durch sphärische Brechkraft (in Dioptrien, dpt), Zylinderkorrektur (in dpt) und Zylinderachse (in Grad) bestimmbar. Als feinste Abstufungen werden für die Brechkraft von Sphäre und Zylinder 0.25dpt, für die Zylinderachse ganzzahlige Gradwerte angegeben.

Wegen der zumindest vom Prinzip her klaren Determiniertheit erscheint es naheliegend, den Vorgang der subjektiven Refraktion von einem Computer ausführen zu lassen. Aus US3,969,020 und US4,105,302 ist daher auch ein Verfahren bekannt, bei dem zunächst eine objektive Refraktion jedes einzelnen Auges und dann eine subjektive Refraktion durch Vorsetzen von Prüfzeichen und Gläsern durch einen Computer gesteuert erfolgt. Nach monokularer Refraktion jedes einzelnen Auges wird abschließend ein binokularer Abgleich durchgeführt. Der Patient erhält akustische Anweisungen von einem Tonband, das ebenfalls von diesem Computer gesteuert wird und gibt Antworten durch Bewegungen eines Steuerknüppels. Obwohl durch dieses bereits 1978 veröffentlichte Verfahren im Prinzip eine vollständige Refraktion durchgeführt werden kann, hat sich die Methode nicht durchsetzen können, vor allem, weil die Bedienung eines Steuerknüppels als "Antworteingabe" bei den gleichzeitig dargebotenen akustischen Fragen und den ebenfalls gleichzeitig dargebotenen Seheindrücken, die im Grenzbereich des Wahrnehmbaren oder Unterscheidbaren liegen, für viele Patienten zu kompliziert ist. Aus demselben Grund hat auch ein aus US4,697,895 bekanntes automatisches Gerät zur Visusprüfung (ohne Brillenglasbestimmung) keine weitere Verbreitung erfahren. Auch hierbei wird ein Steuerknüppel als Antworteinheit verwendet. Auch eine Spracheingabe als Antworteinheit, wie u.a. in EP0319446A1 für ein Gerät zum Screening von Sehstörungen erwähnt, kann das Problem nicht wirklich lösen, denn solange der Patient die Möglichkeit zu freier, akustischer Antwort hat, wird er erfahrungsgemäß immer wieder unerwünschte Antworten geben. So wird er z.B. auf die Frage, ob er mit Glas 1 oder mit Glas 2 die Zahlen deutlicher sehe, statt der erwünschten Antwort "1" oder "2" die Zahlen vorlesen oder bemerken, daß ihm nun sein Auge träne. Andere bekannte Antworteinheiten für Computer wie Tastaturen, Mäuse oder Lightpens, mit denen vorgegebene Antworten auf einem Bildschirm angewählt werden können, sind für den, der an den Umgang damit nicht gewöhnt ist, ebenfalls zu kompliziert.

Schließlich kann auch nicht erwartet werden, daß einmal festgelegte sprachliche Anweisungen und Fragen von allen Patienten gleich gut verstanden werden, auch nicht innerhalb eines Sprach- oder Dialektbereiches, denn auch soziokulturell bedingte Unterschiede in der Sprachauffassung führen erfahrungsgemäß häufig zu Mißverständnissen. In der praktischen Anwendung hat sich gezeigt, daß sogar die Darbietung von Landoldt-Ringen als Sehzeichen, obwohl für augenärztliche Gutachten in der Bundesrepublik Deutschland vorgeschrieben, im Routinebetrieb der Brillenanpassung zu kompliziert und zeitraubend und daher nicht benutzbar ist.

**Aufgabe der vorliegenden Erfindung** ist es, den Arzt von der Routinetätigkeit der Refraktionsbestimmung weitgehend durch computergesteuerte Automatisierung zu befreien, ihm dabei aber die Flexibilität und Lernfähigkeit des menschlich gesteuerten Betriebs so weit wie möglich zu erhalten. Vor allem aber soll dem zu Untersuchenden eine Antwortschnittstelle angeboten werden, die dieser ohne spezielle Vorkenntnisse oder Vorübungen leicht bedienen kann, und die ihm die Entscheidung für eine der jeweiligen Antwortalternativen möglichst leicht macht.

### Kurze Darstellung der Erfindung

Die **Aufgabe** wird **erfindungsgemäß** auf folgende Weise gelöst. Dem Patienten werden Sehzeichen mit Hilfe eines kommerziell verfügbaren Sehzeichenprojektors bzw. Computerbildschirms präsentiert, Gläser mittels eines ebenfalls kommerziell verfügbaren Phoropters vorgesetzt und akustische Fragen und Anweisungen dargeboten. Sehzeichenprojektor, Phoropter und akustische Ausgabe werden von einem Computer entsprechend den bekannten Regeln der Refraktionsbestimmung gesteuert. Gleichzeitig zu den akustischen Fragen werden Antwortalternativen schriftlich oder graphisch im gleichen Gesichtsfeld wie die Sehzeichen dargeboten, so daß der Patient nur mit Augenbewegungen, d.h. bei konstanter Kopfhaltung, zwischen beiden hin- und herschauen kann. Die Einheit, die die Antwortalternativen darbietet, ist oberhalb seines Schoßes angebracht, so daß er gleichzeitig mit ihr seine Hände sehen kann, wenn er diese auf die Antworteinheit auflegt. Er antwortet, indem er eine der Alternativen durch manuelles Drücken auswählt.

Dadurch, daß dem zu Untersuchenden die Antwortalternativen gleichzeitig mit der jeweiligen Frage präsentiert werden, ist die Entscheidung für genau eine dieser Alternativen für ihn nicht nur nicht schwerer, sondern sogar wesentlich leichter als bei der Möglichkeit zu freier, akustischer Antwort, denn er sieht nochmals die bereits gehörten Alternativen und muß sich eindeutig festlegen.

Der Betreiber der Einrichtung hat die Möglichkeit, die Fragen und Anweisungen selbst zu formulieren und sprachlich in das Gerät einzugeben. Außerdem kann er zwischen verschiedenen Verzweigungsmöglichkeiten des Refraktionsablaufes auswählen. Er kann so nach seinen eigenen Lernerfahrungen den Vorgang für sein Patientenkollektiv optimieren.

### Bevorzugte Ausführung und Ausführungsalternativen

Die Präsentation der Sehzeichen erfolgt durch einen Projektor, wobei die Entfernung zwischen Patient und Projektionswand (oder Bildschirm) nach den geltenden Richtlinien 4-6m betragen soll. Die Auswahl der bei den einzelnen Schritten zu projizierenden Sehzeichen (einschließlich der bei manchen Schritten nötigen Polarisations- oder Farbfilter) durch den Computer erfolgt über eine dem Stand der Technik entsprechende digitale Schnittstelle, z.B. seriell nach RS232. Ebenfalls über eine derartige Schnittstelle erfolgt die Auswahl der dem Patienten mittels eines handelsüblichen Phoropters vorgesetzten Linsen (incl. Polarisations- oder Farbfilter oder Lochblenden). Außerdem steuert der Computer über einen Digital-Analog-Wandler einen akustischen Ausgang mit Kopfhörer oder Lautsprecher an, über den dem Patienten die nötigen Anweisungen und Erklärungen gegeben sowie Fragen gestellt werden. Diese Anweisungen, Erklärungen und Fragen sind in digitaler Form im Computer gespeichert. Der Betreiber des Gerätes muß sie über ein Mikrofon und einen Analog-Digital-Wandler akustisch in den Computer eingeben, wobei die genaue Wahl der Formulierung seiner persönlichen Erfahrung und Einschätzung überlassen bleibt. Außerdem kann er sie in gleicher Weise zu einem späteren Zeitpunkt entsprechend seinen Erfahrungen ändern. Er muß sich lediglich an einen vorgegebenen "Musterkatalog" halten, in dem die formal-logische Stellung der jeweiligen Anweisung bzw. Frage festgelegt ist. Ein solcher Musterkatalog ist im folgenden aufgeführt:
1) Schauen Sie bitte durch die beiden Öffnungen auf die Wandtafel. Sie sehen dort Zahlen oder Buchstaben, die Sie genau beobachten müssen. Außerdem sehen Sie in dem kleinen Spiegel oben ihre Hände und die Antworteinheit. Ihnen werden nun verschiedene Brillengläser vorgesetzt und dazu Fragen gestellt. Bitte geben Sie Ihre Antworten durch Drücken der Felder auf der Antworteinheit. Drücken Sie jeweils so, daß die Anzeige kurz verschwindet. Haben Sie die Anleitung verstanden? Dann drücken sie bitte auf "ja". Sonst drücken Sie bitte "nein" oder "wiederholen".
2) Können Sie zumindest einen Teil dieser Buchstaben lesen?
3) Können sie das lesen?
4) Wird das Sehen mit dem nächsten Glas besser?
5) Wird es so besser?
6) Wird das Sehen mit dem nächsten Glas schlechter?
7) Wird es so schlechter?
8) Sehen Sie mit diesem Glas 1 besser?
9) Ist 1 besser?
10) Oder mit diesem Glas 2? Oder sind beide gleich?
11) Oder 2?
12) Sehen Sie jetzt zwei Zahlenreihen übereinander?
13) Vergleichen Sie bitte die beiden Zahlenreihen miteinander! Sie sehen beide nicht ganz scharf. Erscheint Ihnen denn eine von beiden schärfer als die andere? Bemerken Sie einen Unterschied?
14) Ist immer noch eine Reihe schärfer?
15) Ist die obere Zahlenreihe schärfer oder die untere?
16) Ist oben schärfer oder unten?
17) Der Seheindruck kann jetzt schlechter werden, die Buchstaben werden dann undeutlicher.
18) Können Sie vier Zahlen erkennen?
19) Erscheinen Ihnen alle Zahlen gleich deutlich und gleich schwarz?
20) Erscheinen Ihnen die Zahlen im roten und im grünen Feld gleich schwarz?
21) Sind die Zahlen im roten oder im grünen Feld schwärzer?
22) Achten Sie bitte jetzt vor allem auf die Rundungen der Buchstaben! Wir vergleichen jeweils zwei Gläser nacheinander.
23) Wohin zeigt das E?
24) Sind die 9 und die 6 gleich schwarz?
25) Ist die 9 schwärzer oder die 6?
26) Sind die 3 und die 5 gleich schwarz?
27) Ist die 3 schwärzer oder die 5?
28) Die Gläser werden nun schrittweise verändert, bis Sie die Buchstaben nicht mehr klar erkennen können. Versuchen Sie bitte zu lesen, auch wenn es sehr anstrengt!
29) Können Sie die Buchstaben noch lesen?
30) Geht das noch?
31) Sehen Sie durch das kleine Loch schärfer?
32) Schauen Sie bitte nach vorne !
33) Passen Sie nun besonders gut auf!
34) Ich hoffe, Sie können sich noch konzentrieren !
35) Schauen Sie bitte genau hin !
In diesem Musterkatalog sind die Fragen 3,5,7,9,11,14,16 und 30 jeweils Kurzfassungen der vorhergehenden. Sie werden bei unmittelbar aufeinander folgenden Wiederholungen präsentiert. Die Anweisungen 32-35 haben lediglich ermunternden Charakter und werden in wählbarer Häufigkeit zufallsgesteuert an dafür geeigneten Stellen dargeboten.

Der Patient gibt seine Antworten manuell durch Drücken einer von mehreren möglichen Tasten bzw. Feldern einer Antworteinheit, auf der die gerade aktuell gültigen Antwortalternativen schriftlich oder graphisch erkennbar sind. Die über dem Schoß des Patienten montierte Antworteinheit wird ihm bevorzugt durch einen kleinen Hohlspiegel im oberen Teil des vom Phoropter freigegebenen Gesichtsfeldes optisch dargeboten, damit er seinen Kopf zum Antworten nicht bewegen muß, was den Ablauf empfindlich stören würde. Gleichzeitig sieht er so auch seine Hände, die die Einheit bedienen. Durch einen Hohlspiegel von ca. 1.5dpt kann er die Antworteinheit mit der gleichen Akkommodation scharf erkennen wie die in 4-6m dargebotenen Sehzeichen. Allerdings ist die Abbildungsschärfe der Antworteinheit relativ unkritisch, da der Sehwinkel, unter dem die auf ihr präsentierten Buchstaben oder Graphiken erscheinen, sehr groß ist im Vergleich zu denen des Sehzeichenprojektors. Daher kann anstelle des Hohlspiegels auch ein Planspiegel verwendet werden, wenn man eine geringe Unschärfe der Abbildung der Antworteinheit in Kauf nimmt.

Bei der Verwendung eines Spiegels oder einer ungeraden Anzahl von Spiegeln zur Einblendung der Antworteinheit in das Gesichtsfeld des Phoropters muß die schriftliche oder graphische Information auf der Antworteinheit spiegelbildlich dargestellt werden. Dieses wird in anderen Ausführungen der Erfindung vermieden, in denen die optische Abbildung entweder durch ein optisches System mit einer geraden Anzahl von Plan- oder Hohlspiegeln oder durch ein Prismensystem oder durch eine Lichtleiteroptik erfolgt.

Ein zum oben aufgeführten Musterkatalog passender Satz von Antworten wird im folgenden aufgelistet:
1) Ja / Nein
2) Ja / Nein / Wiederholen
3) 1 / 2 / Gleich / Wiederholen
4) Oben / Unten
5) Rot / Grün
6) E / 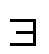 / 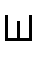 / 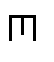
7) 9 / 6
8) 3 / 5
Vorteilhafterweise wird die Antworteinheit als LCD-Display mit aufgelegtem, transparentem Touchpanel ausgelegt. In einer anderen Ausführung mit Tasten wird z.B. auf Taste (1) entweder "ja" oder "1" oder "oben" oder "rot" oder "E" oder "9" oder "3" durch alternatives Ausleuchten oder durch alternative Darstellung mittels LCD-Display oder Leuchtdiodenschrift hervorgehoben.

Der logische Ablauf der subjektiven Refraktion im einzelnen wird in der Literatur auf etwas verschiedene Weise beschrieben. Im Computerprogramm sind daher mehrere Alternativen für die einzelnen Unterabschnitte des Refraktionsablaufes vorgesehen, da der eine Arzt eher das eine, ein anderer eher ein anderes Verfahren vorzieht, und da vor allem die verschiedenen Verfahren für den Patienten unterschiedlich schwierig sind. Da die Verfahren sich teilweise überlappen, ergibt sich eine gewisse Redundanz, die aus Sicherheitsgründen durchaus erwünscht ist. In groben Zügen ist der Ablauf folgendermaßen:
Gestartet wird mit den Ergebnissen einer objektiven oder einer früheren Refraktion, falls vorhanden, und zwar für jedes Auge einzeln. Die Sehzeichen werden zunächst schrittweise verkleinert. Dem Patienten wird dabei die Frage "Können sie das lesen?" gestellt, er hat die Antwortmöglichkeiten "ja" oder "nein". Wenn er mit "nein" antwortet, werden die Sehzeichen um eine Stufe vergrößert, dann werden sphärische Konvexlinsen mit aufsteigender Brechkraft vorgesetzt und der Patient gefragt: "Wird die Sehschärfe schlechter?" mit den Antwortmöglichkeiten "ja", "nein" und "bitte wiederholen". Sobald das Sehen schlechter geworden ist, werden Konkavlinsen mit absteigender Brechkraft vorgesetzt und die Frage gestellt: "wird die Sehschärfe besser?", die Antwortmöglichkeiten bleiben. Wenn die Sehschärfe mit Konkavlinsen deutlich besser wird, werden auch die Sehzeichen wieder verkleinert. Insgesamt wird so die "beste sphärische Linse" ermittelt, für die das Sehen bei größerer Brechkraft schlechter, bei kleinerer nicht besser wird.

Wenn ein Astigmatismus vorliegt, wird zunächst der Achsenabgleich durchgeführt, anschließend wird die Zylinderstärke optimiert. Der Patient erhält folgende Anweisungen: "Das Sehen kann jetzt schlechter werden" und "schauen Sie bitte auf die Rundungen der Zahlen oder Buchstaben". Mit Hilfe der sogenannten "Kreuzzylindermethode", bei der eine Linse vorgesetzt wird, die in zwei zueinander senkrechten Richtungen jeweils einen Astigmatismus von +0.5dpt und -0.5dpt hat, wird zunächst festgestellt, ob überhaupt ein Astigmatismus vorliegt. Wenn dies der Fall ist, wird die Zylinderachse des Patienten iterativ so optimiert, daß sie in der Mitte (d.h. in 45 Grad Abstand) zwischen den beiden Achsen des Kreuzzylinders liegt. Der Seheindruck ist dabei für den Patienten nie optimal, sondern er vergleicht zwei Seheindrücke daraufhin, ob sie gleich (gut oder schlecht) sind. Die Fragen lauten daher "Ist das Sehen in der Stellung 1 besser" - Glaswechsel - "oder in der Stellung 2?", die Antwortmöglichkeiten sind "1", "2", "gleich" und "wiederholen". Die gesuchte Achse ist gefunden, wenn der Seheindruck für beide Stellungen gleich erscheint.

Wenn die Zylinderachse festliegt, kann die Zylinderstärke ebenfalls mit dem Kreuzzylinder optimiert werden, wobei die resultierende mittlere sphärische Brechkraft konstant gehalten werden muß. Die Fragen und Antworten sind die gleichen wie beim Achsenabgleich. Bei höheren Zylinderstärken kann eine Optimierung der Achse zwischenzeitlich nötig werden. Der gesuchte Wert ist gefunden, wenn der Seheindruck für die Stellung 1 des Kreuzzylinders (d.h. 0.25dpt über dem gesuchten Wert) gleich (gut oder schlecht) empfunden wird wie in der Stellung 2 (d.h. 0.25dpt unter dem gesuchten Wert).

Wenn die Refraktion jedes Auges für sich allein bestimmt worden ist, erfolgt der beidäugige Feinabgleich. Eine Möglichkeit hierzu verläuft folgendermaßen. Mit Hilfe von senkrecht zueinander stehenden Polarisationsfiltern sowohl am Sehzeichenprojektor als auch am Phoropter werden dem Patienten zwei Reihen von Sehzeichen gleichzeitig dargeboten. Er hat den Eindruck, beide Reihen mit beiden Augen gleichzeitig zu sehen, sieht jedoch in Wahrheit nur z.B. mit dem rechten Auge die obere, mit dem linken Auge die untere Reihe. Dem Patienten wird die Frage gestellt: "Sehen sie die obere Reihe schärfer oder die untere?" mit den Antwortmöglichkeiten "oben", "unten" oder "nicht unterscheidbar". Für das der unschärferen Reihe entsprechende Auge wird die sphärische Brechkraft um 0.25dpt bis maximal 0.5dpt abgeschwächt und mit der gleichen Frage getestet, ob dadurch der Unterschied zwischen rechtem und linkem Auge verschwindet.

Sowohl der einäugige als auch der beidäugige Abgleich kann im Bereich kleiner Brechkraftänderungen auch mit Hilfe der chromatischen Aberration (in der Literatur als Rot-Grün-Test bekannt) durchgeführt werden. Hierbei wird gefragt, ob Zeichen auf rotem oder grünem Hintergrund schwärzer erscheinen, oder ob beide gleich sind. Angestrebt wird Gleichheit.

Soll zusätzlich zur Refraktion für die Ferne auch eine Refraktion für die Nähe zur Anpassung einer Lesebrille beim älteren Patienten erfolgen, kann dies ebenfalls ohne Intervention des Arztes mit der hier beschriebenen Erfindung erfolgen, wenn der gewünschte Leseabstand bekannt ist. Es muß dann für jedes Auge die Akkommodationsbreite bestimmt werden. Dies erfolgt durch Vorsetzen von zusätzlichen Gläsern mit negativer Brechkraft in zunehmender Stärke so lange, bis der Patient die in der Ferne dargebotenen Sehzeichen nicht mehr erkennen kann, weil er nicht mehr weiter akkomodieren kann. Er wird also jeweils befragt, ob er die Zeichen noch erkennen kann, wobei die Akkomodationsbreite der maximale Zusatz ist, mit dem dies noch möglich ist. Diese Untersuchung muß monokular erfolgen, weil der Patient binokular wegen der physiologischerweise mit der Akkommodation gekoppelten Konvergenz Doppelbilder wahrnehmen würde. Aus der gefundenen Akkomodationsbreite als Minimum oder Mittelwert der beiden Werte für das rechte und linke Auge ergibt sich dann unmittelbar der Nahzusatz für die Lesebrille. Meist wird dieser so gewählt, daß der Patient mit Lesebrille in der Leseentfernung gerade die Hälfte seiner Akkommodationsbreite aufwenden muß. Ob die Hälfte oder ein etwas anderer Wert verwendet wird, liegt im Ermessen des Arztes.

Das Computerprogramm muß neben dem oben grob skizzierten Ablauf auch testen, ob der Patient unlogisch antwortet. So kann es z.B. nicht sein, daß die Sehschärfe durch Erhöhen der Brechkraft nicht schlechter, aber durch Erniedrigen besser wird. Im Fall von unlogischen Antworten wird zunächst die gerade aktuelle Abfragesequenz einschließlich der zugehörigen Anweisungen wiederholt, bei erneut unlogischem Antworten wird der Betreiber (Arzt) optisch (z.B. über ein Computerterminal) oder akustisch informiert. In diesem Fall ist der Patient für die automatische Untersuchung ungeeignet.

Die aufgeführten Einzelschritte der Refraktionsbestimmung sind zum Teil redundant oder überschneiden sich zumindest. Außerdem sind zahlreiche Modifikationen möglich. Aus diesem Grund werden sie von verschiedenen Anwendern nach deren persönlichen Erfahrungen unterschiedlich bewertet und ausgewählt. Diese Auswahl kann auch bei verschiedenen Patienten verschieden sein, denn die Anwendbarkeit hängt wesentlich von den intellektuellen Fähigkeiten des Patienten ab. Es ist daher nicht zu erwarten, daß ein einmalig fest vorgegebenes Ablaufschema auch nur im Durchschnitt optimale Ergebnisse liefert, obwohl das Problem theoretisch klar determiniert ist. Aus diesem Grund muß der betreibende Arzt die Möglichkeit haben, seine "Refraktionsstrategie" auf einfache und flexible Weise zu definieren und zu modifizieren. Dies erfolgt in folgender Weise: Der Computer durchläuft die einzelnen Schritte der Refraktion und bietet immer dann, wenn Alternativen möglich sind, diese dem Arzt zur Entscheidung an, z.B. "Soll bei der monokularen Bestimmung des besten sphärischen Glases ein Rot-Grün-Test erfolgen?" Wenn die Antwort "Ja" ist, wird weiter gefragt "Unterhalb welcher bis dahin erreichten Visusstufe soll mit Rot-Grün getestet werden?" etc. Dabei bedingen die bis zu einer bestimmten Stufe getroffenen Entscheidungen zum Teil die danach noch möglichen mit. Inwieweit dies der Fall ist, wird vom Computerprogramm geprüft. Auf diese Weise wird erreicht, daß vom Computer die logische Konsistenz des gesamten Vorgangs garantiert wird, während der Arzt alle darüberhinaus noch entscheidbaren Modifikationen festlegt.

Die Refraktionsbestimmung mit Hilfe der vorliegenden Erfindung kann prinzipiell in jeder Sprache (bzw. Dialekt) erfolgen. Vorteilhafterweise werden daher sowohl die akustischen Fragen und Anweisungen als auch die sprachabhängigen Antwortalternativen mehrsprachig vorgesehen, so daß der Anwender etwa mit Hilfe eines Computerterminals die jeweils gewünschte Sprache auswählen kann.

## Patentansprüche

1. Einrichtung zum subjektiven Bestimmen von Augengläsern mit
- einem binocularen optischen System, beispielsweise einem Phoropter, zum Vorsetzen verschiedener Augengläser vor die Augen einer zu untersuchenden Person
- einem Sehzeichenprojektor oder Computerbildschirm zur Darbietung von Sehzeichen in festgelegter Entfernung von den Augen der zu untersuchenden Person
- einer akustischen Ausgabeeinheit, über die der zu untersuchenden Person programmgesteuert Erläuterungen und Fragen dargeboten werden
- einer manuell zu bedienenden Antworteinheit, durch deren Bedienung die zu untersuchende Person ihre Antworten auf die gestellten Fragen gibt
- einem Computer, der mit dem optischen System, dem Sehzeichenprojektor, der akustischen Ausgabeeinheit und der Antworteinheit über Schnittstellen verbunden ist und der in bekannter Weise den Ablauf der Sehzeichendarbietung, die Auswahl der Vorsatzgläser und die Abfolge der Fragen und Erläuterungen entsprechend den Antworten der zu untersuchenden Person steuert,
**gekennzeichnet durch**
- eine Anzeigevorrichtung in der Antworteinheit, die die gerade gültigen Antwortalternativen schriftlich oder graphisch anzeigt und
- eine Einblendvorrichtung, die die Antworteinheit einschließlich der sie bedienenden Hände der zu untersuchenden Person optisch in einen Teil des Gesichtsfeldes des optischen Systems einblendet, durch das die zu untersuchende Person hindurch schaut, so daß diese nur durch Hin- und Herschauen mit den Augen, also ohne Kopfbewegungen, zusätzlich zu den dargebotenen Sehzeichen die Antwortalternativen sieht, und dabei ebenfalls sieht, welche davon sie manuell auswählt.

2. Einrichtung nach Anspruch 1, **gekennzeichnet dadurch,** daß eine zusätzliche Schnittstelle zwischen Computer und Betreiber der Anlage vorhanden ist, über die die oben genannten Erläuterungen und Fragen anhand eines Musterkataloges nach individueller Formulierung sprachlich in den Computer eingegeben werden können, und über die Modifikationen des logischen Ablaufs der Augenglasanpassung ausgeführt werden können.

3. Einrichtung nach Anspruch 1.), dadurch **gekennzeichnet**, daß die Einblendvorrichtung einen oder mehrere Planspiegel oder gewölbte Spiegel umfaßt.

4. Einrichtung nach Anspruch 1.), dadurch **gekennzeichnet**, daß die Einblendvorrichtung ein Prismensystem umfaßt.

5. Einrichtung nach Anspruch 1.), dadurch **gekennzeichnet**, daß die Einblendvorrichtung ein Lichtleitersystems umfaßt.

6. Einrichtung nach Anspruch 1.) oder 2.), dadurch **gekennzeichnet**, daß sowohl die akustischen Erläuterungen und Fragen als auch die an eine Sprache gebundenen alternativen Antwortmöglichkeiten, die auf der Anzeigevorrichtung der Antworteinheit schriftlich dargestellt werden, im Computer in mehreren Sprachen oder Dialekten verfügbar und auswählbar sind.

## Claims

1. Device for the subjective determination of eye glasses by
- means of a binocular optical system, for example a phoropter, for the superimposition of various eye glasses in front of the eyes of the person to be examined
- an eye test chart projector or a computer video screen for the presentation of test symbols at a stipulated distance from the eyes of the person to be examined
- an acoustic output unit, through which program-controlled explanations and questions are presented to the person to be examined
- a manually operated answer unit, by operation of which the person to be examined gives his answers to the questions which have been asked
- a computer which is linked to the optical system, the eye chart projector, the acoustic output unit and the answer unit via interfaces, and which controls the sequence of the presentation of test symbols, the selection of superimposed lenses and the sequence of questions and explanations in accordance with the answers given by the person to be examined in the familiar manner.
**characterized by**
- a display apparatus in the answer unit which displays the alternative answers which are valid at the time being in written or graphic form, and
- an image superimposition device which optically superimposes an image of the answer unit, including the hands of the person to be examined in a part of the visual field of the optical system through which the person to be examined looks, so that the said person sees the alternative answers in addition to the test symbols being presented, just by moving his eyes to and fro, thus without any movement of his head, and by this means also sees which of the same he selects manually.

2. Device in accordance with claim 1.), **characterized by the fact** that an additional interface is located between the computer and the operator of the apparatus through which the afore-mentioned explanations and questions can be input into the computer on the basis of a sample catalog with individual linguistic formulation, and through which modifications to the logical sequence of prescribing eye glasses can be implemented.

3. Device in accordance with claim 1.), **characterized by the fact** that the image superimposition device comprises one or more plane mirrors or curved mirrors.

4. Device in accordance with claim 1.), **characterized by the fact** that the image superimposition device comprises a prism system.

5. Device in accordance with claim 1.), **characterized by the fact** that the image superimposition device comprises an optical fiber system.

6. Device in accordance with claim 1.) or 2.), **characterized by the fact** that both the acoustic explanations and questions and also the alternative answer possibilities linked to a language, which are displayed in written form on the display device on the answer unit, are available and selectable in several languages or dialects in the computer.

## Revendications

1. Dispositif servant à déterminer subjectivement des verres correcteurs de la vue, comprenant :
- un système optique binoculaire, par exemple un phoroptre, permettant de disposer devant les yeux de la personne à examiner différents verres correcteurs;
- un projecteur de signes optiques ou un écran d'ordinateur affichant des signes optiques, placé à une certaine distance des yeux de la personne à examiner;
- une unité de sortie acoustique permettant à la personne à examiner d'entendre des explications et des questions commandées par programme;
- une unité de réponse à commande manuelle permettant à la personne à examiner de répondre aux questions posées;
- un ordinateur relié par des interfaces au système optique, au projecteur de signes optiques, à l'unité de sortie acoustique et à l'unité de réponse, et qui commande selon le schéma habituel l'ordre d'apparition des signes, le choix des verres d'essai, l'enchaînement des questions et des explications en fonction des réponses données par la personne à examiner;
**caractérisé** par :
- un dispositif d'affichage intégré dans l'unité de réponse, qui indique chaque fois par écrit ou au moyen d'un graphique les diverses réponses valables, et
- un dispositif d'incrustation sur image qui permet de visualiser simultanément l'unité de réponse et les mains de la personne à examiner dans une partie du champ visuel du système optique à travers lequel la personne à examiner regarde, de sorte qu'elle aperçoit, par un simple mouvement de va-et-vient des yeux - donc sans bouger la tête -, non seulement les signes optiques qui lui sont présentés mais également les propositions de réponse, de même qu'elle peut voir la réponse qu'elle a sélectionnée manuellement.

2. Dispositif selon la revendication 1.), **caractérisé** en ce qu'il existe une interface supplémentaire entre l'ordinateur et l'opérateur de l'installation, qui permet à ceux qui désirent formuler différemment les explications et les questions mentionnées plus haut d'enregistrer oralement sur l'ordinateur en se basant sur un catalogue d'exemples, et qui donne la possibilité de modifier l'ordre logique de passage des verres correcteurs.

3. Dispositif selon la revendication 1.), **caractérisé** en ce que le dispositif d'incrustation sur image comprend un ou plusieurs miroirs plans ou incurvés.

4. Dispositif selon la revendication 1.), **caractérisé** en ce que le dispositif d'incrustation sur image comprend un système à prismes.

5. Dispositif selon la revendication 1.), **caractérisé** en ce que le dispositif d'incrustation sur image comprend un système de fibres optiques.

6. Dispositif selon la revendication 1.) ou 2.), **caractérisé** en ce que tant les explications et les questions acoustiques que les différentes réponses ayant un rapport avec une langue et apparaissant par écrit sur le dispositif d'affichage de l'unité de réponse sont disponibles et peuvent être sélectionnées sur l'ordinateur dans plusieurs langues ou dialectes.
